# EUROPÄISCHE PATENTSCHRIFT

(11) **EP 3 777 720 B1**
(45) Veröffentlichungstag und Bekanntmachung des Hinweises auf die Patenterteilung: **02.10.2024**
(21) Anmeldenummer: 20189463.1
(22) Anmeldetag: 04.08.2020
(51) Int. Cl.: A61B 17/16

(54) **MEDIZINISCHE ANTRIEBSEINHEIT DER HANDHELD-BAUART MIT SENSOREINRICHTUNG UND KICK-BACK-CONTROL**
MEDICAL DRIVE UNIT OF THE HAND HELD TYPE WITH SENSOR DEVICE AND KICK-BACK CONTROL
UNITÉ D'ENTRAÎNEMENT MÉDICALE DE CONCEPTION PORTABLE DOTÉE D'UN DISPOSITIF CAPTEUR ET À CONTRÔLE DE REBOND

(30) Priorität: 05.08.2019 DE 102019121121
(43) Veröffentlichungstag der Anmeldung: 17.02.2021
(73) Patentinhaber: Aesculap AG, 78532 Tuttlingen (DE)
(72) Erfinder: MILLER, Simon, 78652 Deißlingen (DE); ANDRIS, Robin, 78056 Obereschach (DE); FESENMEYER, Tom, 78056 Villingen-Schwenningen (DE)
(74) Vertreter: Winter, Brandl - Partnerschaft mbB

(56) Entgegenhaltungen:
- WO-A1-2019/141536
- US-A1- 2014 166 323
- US-A1- 2014 216 773

## Beschreibung

Die vorliegende Offenbarung betrifft eine medizinische Antriebseinheit der Handheld/Griff-Bauart zur Aufnahme und zum drehenden Antrieb eines medizinischen Werkzeugs im Bereich der operativen Chirurgie.

### Hintergrund der Erfindung

Grundsätzlich befasst sich die Chirurgie mit der operativen Behandlung von Erkrankungen und Verletzungen, wobei verschiedene fachliche Schwerpunkte wie beispielsweise die Gefäßchirurgie oder die Orthopädie und Unfallchirurgie kategorisiert werden. Allen Schwerpunkten gemeinsam ist das Verwenden einer Vielzahl von medizinischen Instrumenten, zur Realisierung einer Operation. Welche medizinischen Instrumente letztlich Verwendung finden, richtet sich dabei nach Eingriffsort und -art, und nach der Funktion des einzusetzenden medizinischen Instruments.

In den meisten operativen Eingriffen im Bereich der Chirurgie werden Instrumente wie Skalpelle, Scheren oder Pinzetten zum Schneiden, Präparieren und Festhalten von Gewebe verwendet. In der Orthopädie und Unfallchirurgie, die sich mit der operativen Behandlung von Erkrankungen und Verletzungen des Bewegungsapparates beschäftigen, werden jedoch im Verlauf einer Operation neben den gängigen medizinischen Instrumenten oftmals zusätzlich diverse Antriebseinheiten wie chirurgische Bohrer, Sägen oder Fräsen benötigt, um beispielsweise Knochen entsprechend bearbeiten zu können.

Vor allem bei der Osteosynthese, die im Allgemeinen mehrere unterschiedliche Operationsverfahren zur Versorgung von frakturierten / gebrochenen oder anderweitig geschädigten Knochen mit dem Ziel einer möglichst schnellen Wiederherstellung der vollen Funktionsfähigkeit des betroffenen Knochens zusammenfasst, sind derartige Antriebseinheiten heutzutage von großer Bedeutung und regelmäßig in Verwendung.

Um beispielsweise einen frakturierten Knochen adäquat zu versorgen, werden bei der Osteosynthese die einzelnen Bruchstücke des betroffenen Knochens in ihre ursprüngliche Form gebracht / Stellung repositioniert und entsprechend stabilisiert. Für die Stabilisierung des Knochens werden dabei zuerst durch Antriebseinheiten wie chirurgische Bohrer oder Fräsen gezielt Löcher an ausgewählte Bereiche des betroffenen Knochens gebohrt bzw. gefräst, um diesen mit Bohrkanälen zu versehen. In diese Bohrkanäle werden dann im Anschluss je nach Verfahren entsprechende Implantate wie zum Beispiel Schrauben eingesetzt, um den betroffenen Knochen zu fixieren.

Während der Anwendung solcher chirurgischen Antriebsmaschinen am Patienten kann es jedoch vorkommen, dass das an der medizinischen Antriebseinheit eingespannte drehbare, chirurgische Werkzeug im Knochen des Patienten blockiert / verkantet. Infolgedessen wird eine Rotationsenergie der medizinischen Antriebseinheit in Richtung des Anwenders generiert, die sich an diesem abbaut. Der Grund dafür liegt oftmals in der Steuerung des Motors der medizinischen Antriebseinheit. Wird der Motor der medizinischen Antriebseinheit zum Beispiel über einen Schiebregler betrieben, welcher drehzahlgeregelt ist, wird das Drehmoment der handgehaltenen medizinischen Antriebseinheit nicht begrenzt, wodurch die der medizinischen Antriebseinheit entgegengebrachte Kraft des Anwenders ab einem gewissen Punkt nicht mehr ausreicht und der Anwender die Kontrolle über die medizinische Antriebseinheit verliert.

Das aus diesem Kontrollverlust des Anwenders resultierende Drehen der Antriebseinheit in die Bewegungsrichtung des eingespannten Werkzeugs, was auch als ausschlagende Bewegung der medizinischen Antriebseinheit bezeichnet wird, kann sowohl zu Verletzungen des Anwenders, vor allem im Armbereich und an Hand- und Schultergelenken, als auch zu Verletzungen des Patienten führen, die wiederum Einfluss auf den Verlauf der Operation haben können.

### Stand der Technik

Aus dem Stand der Technik sind medizinische Antriebseinheiten der Handheld-Bauart zur Aufnahme chirurgischer Werkzeuge bekannt, die anhand einer Drehmomentbegrenzung gewährleisten sollen, dass vorstehend beschriebene anwendungsbezogene Nachteile vermieden werden.

Eine derartige medizinische Antriebseinheit sieht eine integrierte Drehmomentbegrenzung vor, die auf einem Anstieg des Stroms in Abhängigkeit von der Zeit basiert. Kommt es bei der medizinischen Antriebseinheit also zu einem steilflankigen Anstieg des Stroms in einer bestimmten Zeit, wird dies auf eine Blockade oder Bewegung der Antriebseinheit in die Rotationsrichtung des eingespannten Werkzeugs zurückgeführt.

Allerdings wird bei einer derartigen Drehmomentbegrenzung kein direktes Blockieren des Werkzeugs oder eine außer Kontrolle des Anwenders geratene Bewegung der medizinischen Antriebseinheit erfasst / bestimmt. Stattdessen wird lediglich ein Überschreiten eines vor der Anwendung eingestellten maximalen Drehmoments über das Erkennen der Stromänderungsgeschwindigkeit (di/dt) erfasst. Ein vor einer Anwendung eingestelltes maximales Drehmoment kann den Anwender außerdem weiterhin in seiner Anwendung der medizinischen Antriebseinheit einschränken, da sich das max. Drehmoment nicht an die sich während der Anwendung ggf. verändernden Gegebenheiten anpassen kann. Des Weiteren gestaltet sich die Realisierung der vorstehend beschriebenen Drehmomentbegrenzung im Zusammenhang mit den an ein Medizinprodukt gestellten Anforderungen, zum Beispiel in Hinsicht auf Aufbereitungsprozesse, als eher schwierig. Schließlich besteht die Möglichkeit, dass die Antriebseinheit während eines chirurgischen Eingriffs von unterschiedlichen Personen benutzt wird, die unterschiedliche Körperkräfte entwickeln können. Dies bedeutet, dass ein ggf. vorab eingestelltes maximales Drehmoment für die eine Person zu klein oder zumindest akzeptabel ist, um die Antriebseinheit sicher halten zu können, wohingegen für die andere Person das maximale Drehmoment zu hoch eingestellt ist, was dazu führen kann, dass die Antriebseinheit bei Annäherung dieses maximalen Drehmoments unkontrollierte Bewegungen in der Haltehand dieser Person ausführen kann. Zur Vermeidung dieser kritischen, weil unkontrollierten Bewegungssituation müsste bei einem Übergeben der Antriebseinheit von einer Person zu einer anderen Person jedes Mal das maximal zulässige Drehmoment neu eingestellt werden, was sehr aufwendig und unzuverlässig ist.

US 2014/166323 A1 offenbart eine Vorrichtung und Verfahren zum Schützen einer Bedienungsperson vor plötzlichen, unerwarteten oder gefährlichen Bewegungen von angetriebenen handgehaltenen Werkzeugen.

US 2014/216773 A1 offenbart ein Verfahren zum Steuern einer Elektrowerkzeugmaschine mit einem Elektromotor zum Antreiben eines rotierenden Einsatzwerkzeugs, wobei die Elektrowerkzeugmaschine in einem von mehreren Betriebsmodi betrieben und mindestens ein Parameter der Werkzeugmaschine erfasst wird.

### Allgemeine Beschreibung der vorliegenden Offenbarung

Angesichts der vorstehend erläuterten Problematik besteht daher die grundsätzliche Aufgabe der vorliegenden Offenbarung darin, eine medizinische Antriebseinheit vorzugsweise der Handgriff-/Handheld-Bauart bereitzustellen, die eine reibungslose Anwendung der medizinischen Antriebseinheit ermöglicht, um Verletzungen und eine außer Kontrolle des Anwenders geratene Anwendung zu vermeiden und den gesamten Operationsverlauf dadurch im Wesentlichen sicherer zu machen.

Diese Aufgabe wird durch eine medizinische Antriebseinheit vorzugsweise der Handgriff-/Handheld-Bauart mit den Merkmalen des Patentanspruchs 1 gelöst. Vorteilhafte Ausgestaltungen der Offenbarung sind Gegenstand der Unteransprüche.

Der Kerngedanke der vorliegenden Offenbarung besteht demzufolge darin, nicht oder nicht nur ein ggf. einstellbares maximal zulässiges Drehmoment an dem Werkzeug zu erfassen / zu überwachen / zu regeln, wie dies im Stand der Technik vorgeschlagen wird, sondern (als alleinige oder zusätzliche Maßnahme) Bewegungen der Antriebseinheit, bzw. (vorzugsweise von der Antriebseinheit ausgelöste) Haltebewegungen der Benutzerperson zu detektieren / bestimmen, um aus diesen Bewegungen bzw. (daraus ermittelten) Bewegungsbeträgen Rückschlüsse auf die aktuelle Bewegungssituation (und damit indirekt auf die Drehmoment-/ Kraftbeaufschlagung der Antriebseinheits-Halterung, bzw. der Haltehand der Benutzerperson durch die Antriebseinheit) zu ziehen und basierend hierauf den Antrieb ggf. hoch- und / oder zurückzufahren.

In anderen Worten ausgedrückt bewirkt ein Überschreiten einer durch eine Benutzerperson maximal aufbringbaren Haltekraft zwangsläufig ein sogenanntes Nachgeben der Haltehand und damit eine entsprechende (unkontrollierte) Bewegung der Antriebseinheit. Wird dieses Nachgeben der Haltehand erfasst / erkannt, kann hieraus auf ein für die aktuelle Benutzerperson zu hohes Antriebsdrehmoment geschlossen und demzufolge die Antriebseinheit zurückgefahren werden. Damit wird die Handhabung einer derart geregelten Antriebseinheit sicherer und man spart sich obendrein ein Abspeichern von mehreren unterschiedlichen maximalen Drehmomenten für unterschiedliche Bedienerpersonen.

So ist im Konkreten eine medizinische Antriebseinheit der Handheld-Bauart vorgesehen, die dazu ausgelegt ist, ein medizinisches, vorzugsweise chirurgisches Werkzeug aufzunehmen und dieses drehbar mit Hilfe eines Motors zu betreiben, und die gemäß der Erfindung mit einer Sensoreinrichtung versehen ist, welche entweder direkt oder indirekt eine Bewegung der Antriebseinheit oder eine Haltebewegung eines Anwenders erfasst / bestimmt, wobei die Sensoreinrichtung dabei durch die von dem Motor auf das Werkzeug abgegebene Leistung ausgelöst wird. Zudem erweist es sich als vorteilhaft, wenn die medizinische Antriebseinheit der Handheld-Bauart in Kombination mit der Sensoreinrichtung eine integrierte Kick-Back-Control (Rückkopplungssteuerung) aufweist, welche bei Erfassung einer vorbestimmten Antriebseinheits- oder Haltebewegung oder eines vorbestimmten Antriebseinheits- oder Haltebewegungsbetrags eine Reduktion der (aktuellen) Antriebsleistung und/oder ein Abschalten des Motors automatisch bewirkt.

Gemäß einem weiteren Aspekt der Offenbarung ist es vorteilhaft, die Sensoreinrichtung als Inertialsensor und dabei vorzugsweise als Gyroskop und/oder Beschleunigungssensor zu gestalten / vorzusehen, um über drei Achsen (X, Y, Z) eine Bewegung der medizinischen Antriebseinheit in die Bewegungsrichtung des in die medizinische Antriebseinheit eingespannten Werkzeugs detektieren zu können. Dabei können die Bewegungen der medizinischen Antriebseinheit eine Rotationsbewegung oder Richtungsbewegung beschreiben, wobei erstere beispielsweise durch einen Bohrer (Fräser, Acetabulum, Schrauber) und letztere beispielsweise durch eine Sagittalsäge oder Stichsäge ausgelöst wird.

Kommt es während der Anwendung zu einem Blockieren oder einer Blockiertendenz der medizinischen Antriebseinheit, bedingt dies einen Drehmomentanstieg, wodurch sich die Antriebseinheit bei zu geringer entgegengesetzter Kraft des Halters/des Anwenders in die Bewegungsrichtung des eingespannten Werkzeugs bewegt. In Anlehnung daran wird nach einem Aspekt der Erfindung die medizinische Antriebseinheit bereitgestellt, die eine Auswerteelektronik aufweist, welche anhand der Änderungsrate der Bewegung der medizinischen Antriebseinheit in die Bewegungsrichtung des in die medizinische Antriebseinheit eingespannten Werkzeugs detektiert/bestimmt, ob ein erhöhtes Drehmoment vorliegt.

Ein anderer Aspekt sieht die Bereitstellung einer medizinischen Antriebseinheit der Handheld-Bauart vor, wonach der Inertialsensor und die Auswerteelektronik in einem Akku, vorzugsweise im Akkukopf, integriert sind, der weiter vorzugsweise in einen (Hand-)Griff der Antriebseinheit ggf. entnehmbar eingesetzt ist. Durch eine derartige Anordnung bzw. Erweiterung des Akkus und die Möglichkeit den Akku der medizinischen Antriebseinheit und dementsprechend auch den Inertialsensor und die Auswerteelektronik zu entnehmen und / oder auszutauschen, ist es möglich, den Anforderungen an Medizinprodukte, zum Beispiel in Bezug auf Aufbereitungsprozesse, weiterhin gerecht zu werden. Des Weiteren wirkt sich dies positiv auf die Lebensdauer und die Betriebskosten der medizinischen Antriebseinheit aus, und ermöglicht eine relativ einfache Realisierung des Systems, da der Inertialsensor in eine bereits bestehende Steuerung implementiert / integriert wird.

Für eine konstruktive Umsetzung des offenbarten Prinzips ist es vorteilhaft im Gegensatz zum bisherigen Stand der Technik von einer Konfiguration des Drehmoments vor Anwendung der medizinischen Antriebseinheit abzusehen. Dies ermöglicht, dass die Drehmomentbegrenzung der medizinischen Antriebseinheit der Handheld-Bauart keine externe Recheneinheit oder Datenspeicherung / - auswertung benötigt und autark ohne weitere Geräte und drahtlose oder kabelgebundene Kommunikation funktioniert.

Gemäß einem anderen Aspekt der Offenbarung ist es weiterhin vorgesehen, eine Drehmomentbegrenzung der medizinischen Antriebseinheit durch die Steuer- oder Regeleinrichtung permanent adaptierbar auf die Anwendung und den Anwender zuzuschneiden, wobei ein Drehmoment der medizinischen Antriebseinheit durch die Steuer- oder Regeleinrichtung an das für den Anwender maximal verwendbare Drehmoment angepasst wird, um ein Auftreten eines zu hohen Drehmoments für den Anwender im Verlauf der Anwendung zu vermeiden.

In anderen Worten ausgedrückt, hat die vorgesehene Steuer- oder Regeleinrichtung eine (zusätzliche) Lerneinheit/Lernfunktion, welche für die jeweilige, vorzugsweise aktuelle Anwendung und/oder den jeweiligen, vorzugsweise aktuellen Anwender jenes (individuelle) Drehmoment identifiziert (beispielsweise im Laufe einer Anwendung), bei welchem eine durch die Antriebseinheit (infolge des aktuell auftretenden Drehmoments am Werkzeug) erzeugte/induzierte Antriebseinheitsbewegung/Antriebseinheitsbeschleunigung (bzw. Handbewegung/Handbeschleunigung) einen (vor-)bestimmten Betrag erreicht/überschreitet und dieses identifizierte Drehmoment als das (aktuell) maximal zulässige Drehmoment (temporär) festlegt.

Im weiteren Verlauf der Anwendung, wird der Drehmomenteintrag der Antriebseinheit in das Werkzeug so begrenzt (beispielsweise durch Einstellen einer Rutschkupplung zwischen Antrieb und Werkzeug), dass dieses maximal zulässige Drehmoment dann nicht mehr überschritten wird.

Auf diese Weise kann beispielsweise das (aktuell) maximal zulässige Drehmoment nicht nur einem bestimmten Anwender und/oder einer bestimmten Anwendung zugeordnet werden, sondern es könnte sich beispielsweise auch der aktuellen Konstitution des Anwenders, d.h. der fortschreitenden Ermüdung des Anwenders anpassen, wonach die Drehmomentbelastung des allmählich ermüdenden Anwenders (kontinuierlich) abnimmt. Letzteres ließe sich technisch dadurch erreichen, indem trotz erstmaligem Festlegens des maximal zulässigen Drehmoments beispielsweise durch eng getaktete Messzyklen die aktuellen Bewegungs-Beschleunigungsbeträge immer wieder erfasst, mit dem (vor-)bestimmten Betrag verglichen und das aktuelle, maximal zulässige Drehmoment entsprechend verifiziert (ggf. verringert) wird (feed-back-control). Alternativ wäre es aber auch denkbar, beispielsweise eine durchschnittliche Ermüdungskurve zu speichern, entlang dieser das aktuelle, maximal zulässige Drehmoment ohne weitere Messzyklen verändert (verringert) wird (feed-forwardcontrol).

Dabei hängt eine Länge der Drehmomentbegrenzung der medizinischen Antriebseinheit nach einem weiteren Aspekt der Offenbarung von der Zeit ab, die der Anwender für eine Stabilisierung der medizinischen Antriebseinheit benötigt.

Schließlich betrifft ein anderer Aspekt der Offenbarung den in der medizinischen Antriebseinheit untergebrachten Motor, welcher als ein kommutierter Elektromotor vorgesehen ist. Dabei ist es gemäß einem weiteren Aspekt vorteilhaft, dass die Kick-Back-Control bei einer Erfassung einer vorbestimmten Haltebewegung oder eines vorbestimmten Haltebewegungsbetrags ein Abschalten und / oder Abbremsen und / oder ein Ansteuern des Motors in entgegenwirkende Richtung automatisch bewirkt.

An dieser Stelle sei ausdrücklich darauf hingewiesen, dass die vorstehenden Aspekte einzeln sowie in beliebiger Kombination miteinander die gestellte Aufgabe lösen können und daher einzeln oder in beliebiger Kombination im Rahmen dieser Anmeldung beanspruchbar sein sollen.

### Kurzbeschreibung der Figuren

Fig. 1 zeigt eine Seitenansicht einer medizinischen Antriebseinheit der Handheld-Bauart zur Veranschaulichung eines Systemaufbaus gemäß einem Ausführungsbeispiel der Offenbarung, und
Fig. 2 zeigt ein Flussdiagramm für einen schematischen Ablauf der Realisierung einer Kick-Back-Control gemäß dem in Fig. 1 dargestellten Ausführungsbeispiel.

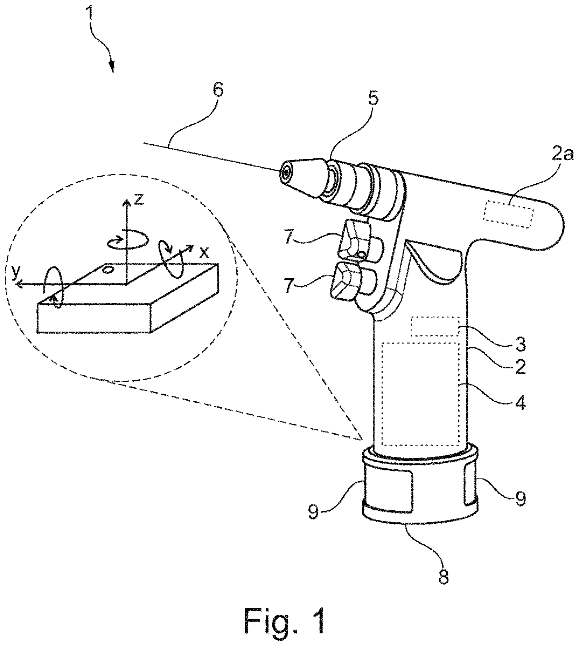

Fig. 1 zeigt ein Ausführungsbeispiel einer medizinischen Antriebseinheit 1 (medizinisches Instrument) der Handheld-Bauart, welche einen Griff 2, einen Motor 2a, eine Sensoreinrichtung und Auswerteelektronik 3 (diese sind in Fig.1 als eine Einheit dargestellt, können aber auch getrennte Einheiten sein), einen Akku 4, einen Getriebeaufsatz 5, welcher als Bohrgetriebeaufsatz ausgebildet ist, ein in den Getriebeaufsatz 5 eingespanntes medizinisches Werkzeug 6, welches als Bohrwerkzeug ausgebildet ist, zwei Bedienungselemente 7, eine Abdeckung / ein Cover 8 und zwei Abdeckungsfreigabeknöpfe 9 aufweist.

Die in Fig. 1 angedeutete Sensoreinrichtung 3, die in Form eines Inertialsensors, und vorzugsweise als Gyroskop und / oder Beschleunigungssensor, ausgebildet ist und über drei Achsen eine Bewegung der medizinischen Antriebseinheit 1 erfasst, sowie die zur Auswertung der von der Sensoreinrichtung 3 erfassten Daten benötigte Auswerteelektronik (Steuer-/Regeleinrichtung) 3 sind im Inneren des Griffs 2 unterhalb eines in Fig. 1 angedeuteten Akkus 4 oder direkt im Akkukopf vorgesehen. Der sich im Inneren des Griffs 2 befindliche Akku 4 ist dabei derart gestaltet, dass er beispielsweise zum Austauschen oder für Aufbereitungsprozesse der medizinischen Antriebseinheit 1 durch Abnehmen der Abdeckung 8 vom Griff 2 entnehmbar ist.

Auch eine in Fig. 1 nicht bzw. nur zusammen mit der Sensoreinheit 3 dargestellte Steuer- oder Regeleinrichtung, zum Betreiben des Motors 2a, ist im Akkukopf bzw. innerhalb des Griffs 2 vorgesehen. Zudem ist der in Fig. 1 angedeutete Motor 2a, der als kommutierter Elektromotor ausgebildet ist und welcher von dem Akku 4 mit elektrischen Strom versorgt wird, im Inneren der medizinischen Antriebseinheit 1 untergebracht. Eine derartige Anordnung der einzelnen Komponenten hat den Vorteil, dass die medizinische Antriebseinheit 1 autark ohne weitere Geräte und drahtlose oder kabelgebundene Kommunikation funktionieren kann.

Generell wird die medizinische Antriebseinheit 1 durch eine von dem Motor 2a generierte Leistung / Kraft angetrieben, wobei der Motor 2a über die Steuer- oder Regeleinrichtung in seiner Leistung regulierbar ist. Über eine in Fig. 1 nicht dargestellte Kupplung wird die von dem Elektromotor erzeugte Leistung auf den Getriebeaufsatz 5 übertragen. Die von der Kupplung übertragene Leistung des Elektromotors wird wiederum von dem in dem Getriebeaufsatz 5 integrierten Getriebe in ein Drehmoment umgewandelt, welches genutzt wird, um das in Fig. 1 als Bohrwerkzeug ausgebildete eingespannte medizinische Werkzeug 6 drehend anzutreiben.

Der Griff 2 der medizinischen Antriebseinheit 1 ist ergonomisch gestaltet, wobei der Griff 2 ein zylinderförmiges Handstück und eine an einem distalen Ende des Griffs 2 vorhandene Ausbuchtung zum Ablegen des Daumens der von dem Anwender zur Steuerung der medizinischen Antriebseinheit 1 genutzten Hand aufweist. An dem distalen Ende geht der Griff 2 einstückig in eine zigarrenförmige Erweiterung über, die sich in einer Querrichtung des Griffs 2 erstreckt. Der Durchmesser der zigarrenförmigen Erweiterung ist dabei kleiner als der Durchmesser des zylinderförmigen Handstücks des Griffs 2 und reicht aus, den Motor 2a darin aufzunehmen.

An einem distalen Ende der zigarrenförmigen Erweiterung ist der Getriebeaufsatz 5 in die medizinische Antriebseinheit 1 eingespannt bzw. darin gelagert, wobei der Getriebeaufsatz 5 je nach Anwendung austauschbar ist. In den Getriebeaufsatz 5 wird wiederum je nach Getriebeaufsatztyp ein passendes medizinisches Werkzeug 6 (Bohrer, Fräser, etc.) eingespannt, welches ebenfalls austauschbar ist.

In einem distalen (dem Patienten zugewandten) Bereich des zylinderförmigen Handstücks des Griffs 2 sind die zwei Bedienungselemente 7 vorgesehen. Durch Betätigen der Bedienungselemente 7 durch den Anwender wird das in den Getriebeaufsatz 5 der medizinischen Antriebseinheit 1 eingespannte medizinische Werkzeug 6 bedient / betrieben. Dabei können die Bedienungselemente 7 in Bezug auf verschiedene Parameter, die für die Bedienung des in den Getriebeaufsatz 5 der medizinischen Antriebseinheit 1 eingespannten medizinischen Werkzeugs 6 von Nutzen sein können, wie zum Beispiel die Drehrichtung oder Drehgeschwindigkeit konfiguriert sein.

An einem proximalen (dem Anwender zugewandten) Ende des Griffs 2 ist die Abdeckung 8 in Form einer Kappe vorgesehen, die die medizinische Antriebseinheit 1 an einer Stirnseite des zylinderförmigen Handstücks des Griffs 2 verschließt. In der Abdeckung 8 sind zwei Abdeckungsfreigabeknöpfe 9 integriert, durch deren Betätigung die Abdeckung 8 abgenommen werden kann.

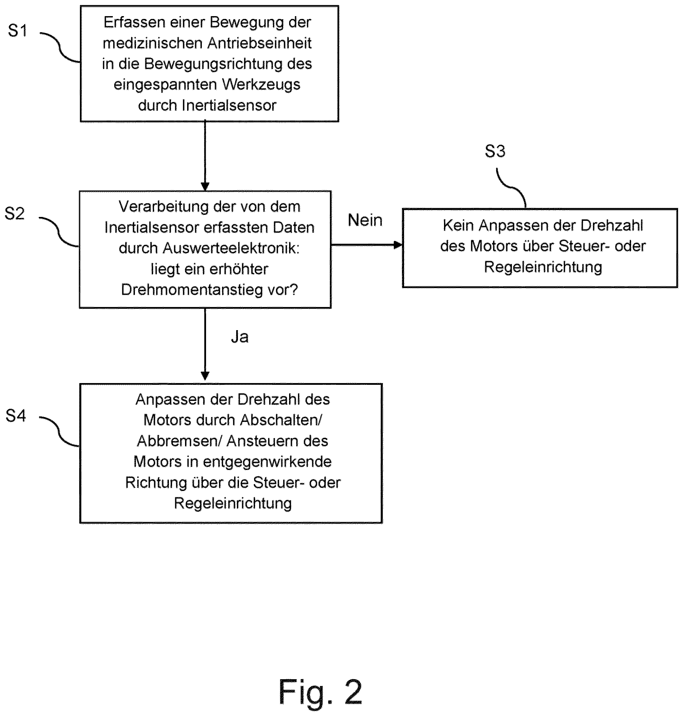

Fig. 2 zeigt ein Flussdiagramm für einen schematischen Ablauf der Realisierung einer Kick-Back-Control gemäß dem in Fig. 1 dargestellten Ausführungsbeispiel.

Kommt die in Fig. 1 dargestellte medizinischen Bohr- / Fräsmaschine 1 beispielsweise während einer Hüftoperation zum Einsatz, muss der Anwender zum Ausschaben des Hüftgelenks (der Hüftgelenkschale) aufgrund der großen Auflagefläche und Festigkeit des Hüftknochens eine sehr hohe Kraft aufwenden, um ein Rotieren der medizinischen Bohr- /Fräsmaschine 1 in die Bewegungsrichtung des in die medizinische Bohr- / Fräsmaschine 1 eingespannten Acetabulum-Werkzeugs 6 zu verhindern.

An dieser Stelle sei darauf hingewiesen, dass das Werkzeug 6 in Fig. 1 nur symbolisch als eine Linie dargestellt ist und nicht die konkrete Form des tatsächlich aktuell eingesetzten Werkzeugs 6 widergegeben wird.

Während einer derartigen Anwendung (z.B. Ausfräsung) sind Drehmomentspitzen infolge eines Blockierens oder einer Blockiertendenz des Werkzeug 6 zu erwarten, wobei diese Drehmomentspitzen in die medizinische Antriebseinheit 1 und von dort in die Haltehand/Arm des Anwenders weitergegeben werden. Demzufolge erfasst der Inertialsensor 3, der vorzugsweise als Gyroskop und / oder Beschleunigungssensor ausgebildet ist, in Schritt S1 vorzugsweise permanent/eng getaktet über drei Achsen (X, Y, Z) hinweg eine Bewegung/Beschleunigung der medizinischen Bohr- / Fräsmaschine 1 insbesondere in die Bewegungsrichtung des in die medizinische Bohr-/ Fräsmaschine 1 eingespannten Acetabulum-Werkzeugs 6.

Die mit dem Inertialsensor 3 verknüpfte Auswerteelektronik 3 verarbeitet in Schritt S2 die von dem Inertialsensor 3 erfassten Bewegungs-/ oder Beschleunigungsdaten der medizinischen Bohr- / Fräsmaschine 1 und ermittelt beispielsweise anhand der Änderungsrate der erfassten Bewegungs-/Beschleunigungsdaten der medizinischen Bohr- / Fräsmaschine 1, ob ein erhöhter / zu hoher Drehmomentanstieg vorliegt bzw. ob das aktuell am Werkzeug 6 anliegende Drehmoment zu hoch ist oder von der haltenden Person kompensiert werden kann. Ermittelt die Auswerteelektronik 3 keinen erhöhten Drehmomentanstieg bzw. kein erhöhtes Drehmoment, d.h. die medizinische Bohr-/ Fräsmaschine 1 kann von der Person ruhig gehalten werden, passt eine Steuer- oder Regeleinrichtung in Schritt S3 die Drehzahl des Motors 2a und / oder das Drehmoment des in die medizinische Bohr- / Fräsmaschine 1 eingespannten Acetabulum-Werkzeugs 6 nicht an, sodass das in die medizinische Bohr- / Fräsmaschine 1 eingespannte Acetabulum-Werkzeug 6 weiterhin mit dem gleichen oder ggf. zunehmenden Drehmoment betrieben wird. Ermittelt die Auswerteelektronik 3 jedoch einen erhöhten / zu hohen Drehmomentanstieg bzw. ein zu hohes Drehmoment, d.h. die medizinische Bohr- / Fräsmaschine 1 kann von der Person nicht ruhig gehalten werden, passt die Auswerteelektronik/Steuer- oder Regeleinrichtung 3 in Schritt S4 die Drehzahl des Motors 2a und / oder das Drehmoment des in die medizinische Bohr- / Fräsmaschine 1 eingespannten Acetabulum-Werkzeugs 6 an, sodass das in die medizinische Bohr-/ Fräsmaschine 1 eingespannte Acetabulum-Werkzeug 6 mit einem geänderten / korrigierten Drehmoment betrieben wird. Das Anpassen/Reduzieren der Drehzahl / des Drehmoments des Motors 2a kann dabei durch Abschalten und / oder Abbremsen und / oder Ansteuern des Motors 2a in entgegenwirkende Richtung oder durch Ändern der Drehmoment-Übertragungsfähigkeit der Kupplung (einstellbare Rutschkupplung) zwischen Motor 2a und Getriebeaufsatz 5 realisiert werden.

Gemäß der vorliegenden Offenbarung ist es zudem vorgesehen, dass die Auswerteelektronik/Steuer- oder Regeleinrichtung 3 lernfähig ist, derart, dass die Steuer- oder Regeleinrichtung 3 die bereits (erstmalig) angepasste Drehzahl oder das bereit (erstmalig) angepasste Drehmoment für die aktuelle Anwendung und/oder den aktuellen Anwender als maximal zulässige Obergrenze beibehält, die im weiteren Anwendungsverlauf nicht mehr überschritten wird. Auf diese Weise kann eine erneute unzulässige Bewegung/Beschleunigung der Antriebseinheit 1 infolge eines ggf. zu hohen Drehmomenteintrags von vornherein vermieden werden.

Eine derartige permanent variable, d.h. sich verändernden Bedingungen (z.B. infolge zunehmender Ermüdung des Anwenders) sich fortlaufend anpassenden / selbsttätige Drehmomentbegrenzung für die weitere Anwendung ermöglicht es dem Anwender, die medizinische Bohrmaschine 1 mit dem für den Anwender maximal verwendbaren Drehmoment zu bedienen, sodass das Hüftgelenk mit dem über die Anwendung hinweg immer maximal möglichen Vortrieb ausgeschabt werden kann, ohne dass manuelle Einstellungen des max. Drehmoments vorgenommen werden müssen.

Zusammenfassend betrifft die Erfindung folglich eine medizinische Antriebseinheit 1 der Handheld-Bauart zur Aufnahme und zum drehenden Antrieb eines medizinischen, vorzugsweise chirurgischen Werkzeugs 6 mittels eines in der Antriebseinheit 1 untergebrachten Motors 2a, vorzugsweise Elektromotors, der über eine Steuer- oder Regeleinrichtung betreibbar ist. Erfindungsgemäß weist eine derartige medizinische Antriebseinheit 1 der Handheld-Bauart eine Sensoreinrichtung 3 zur direkten oder indirekten Erfassung oder Bestimmung einer Bewegung der Antriebseinheit 1 oder einer Haltebewegung eines Anwenders, welche durch den Motor 2a entsprechend seiner auf das Werkzeug 6 abgegebenen Leistung ausgelöst wird und eine Kick-Back-Control auf, die bei Erfassung einer vorbestimmten Antriebseinheits- oder Haltebewegung oder eines vorbestimmten Antriebseinheits- oder Haltebewegungsbetrags eine Reduktion der Antriebsleistung oder ein Abschalten des Motors 2a automatisch bewirkt.

### Bezugszeichenliste

- 1: medizinische Antriebseinheit
- 2: Griff
- 2a: Motor
- 3: Sensoreinrichtung und Auswerteelektronik (Steuer-/Regeleinrichtung)
- 4: Akku
- 5: Getriebeaufsatz
- 6: medizinisches Werkzeug
- 7: Bedienungselement
- 8: Abdeckung / Cover
- 9: Abdeckungsfreigabeknopf

## Patentansprüche

1. Medizinische Antriebseinheit (1) vorzugsweise der Handheld-Bauart zur Aufnahme und zum drehenden Antrieb eines medizinischen, vorzugsweise chirurgischen Werkzeugs (6) mittels eines in der Antriebseinheit (1) untergebrachten Motors (2a), vorzugsweise Elektromotors, der über eine Steuer- oder Regeleinrichtung betreibbar ist, mit einer Sensoreinrichtung (3) zur direkten oder indirekten Erfassung oder Bestimmung einer Bewegung oder Beschleunigung der Antriebseinheit (1) oder einer Haltebewegung oder Haltebeschleunigung eines Anwenders, welche durch den Motor (2a) entsprechend seiner auf das Werkzeug (6) abgegebenen Leistung ausgelöst wird und mit einer Kick-Back-Control, die bei Erfassung einer vorbestimmten Antriebseinheits- oder Haltebewegung/-beschleunigung oder eines vorbestimmten Antriebseinheits- oder Haltebewegungs-/beschleunigungsbetrags eine Reduktion der Antriebsleistung oder ein Abschalten des Motors (2a) automatisch bewirkt, **dadurch gekennzeichnet, dass** die Steuer- oder Regeleinrichtung eine Lernfunktion hat, welche für eine aktuelle Anwendung und/oder einen aktuellen Anwender jenes aktuelle Drehmoment identifiziert und das aktuelle identifizierte Drehmoment als maximal zulässiges Drehmoment festlegt, bei welchem die Kick-Back-Control die Reduktion der Antriebsleistung oder das Abschalten des Motors (2) bewirkt hat, wobei Drehmomente in weiterem Verlauf der Anwendung auf das maximal zulässige Drehmoment begrenzt werden.

2. Medizinische Antriebseinheit (1) der Handheld-Bauart nach Anspruch 1,
**dadurch gekennzeichnet, dass** die Sensoreinrichtung (3) ein Inertialsensor ist, vorzugsweise ein Gyroskop und / oder ein Beschleunigungssensor, welcher über drei Achsen (X, Y, Z) eine Bewegung der medizinischen Antriebseinheit (1) in die Bewegungsrichtung des in die medizinische Antriebseinheit (1) eingespannten medizinischen Werkzeugs (6) erfasst.

3. Medizinische Antriebseinheit (1) der Handheld-Bauart nach einem der Ansprüche 1 oder 2, **dadurch gekennzeichnet, dass** die Steuer- oder Regeleinrichtung anhand der Änderungsrate der Bewegung der medizinischen Antriebseinheit (1) in die Bewegungsrichtung des in die medizinische Antriebseinheit (1) eingespannten medizinischen Werkzeugs (6) ermittelt, ob ein erhöhter Drehmomenteintrag oder ein erhöhter Drehmomentanstieg vorliegt.

4. Medizinische Antriebseinheit (1) der Handheld-Bauart nach Anspruch 2 und 3,
**dadurch gekennzeichnet, dass** der Inertialsensor und die Steuer- oder Regeleinrichtung in einem Griff (2) der Antriebseinheit (1) oder in einem Akku (4) vorzugsweise im Akkukopf integriert sind, der weiter vorzugsweise entnehmbar in den Griff (2) der Antriebseinheit (1) eingesetzt ist.

5. Medizinische Antriebseinheit (1) der Handheld-Bauart nach Anspruch 3,
**dadurch gekennzeichnet, dass** die Drehmomentbegrenzung der medizinischen Antriebseinheit (1) durch die Steuer- oder Regeleinrichtung permanent adaptierbar auf die Anwendung und den Anwender zugeschnitten ist, wobei ein Drehmoment der medizinischen Antriebseinheit (1) durch die Steuer- oder Regeleinrichtung an das für den Anwender aktuell maximal verwendbare Drehmoment vorzugsweise kontinuierlich angepasst ist oder wird, um ein Auftreten eines zu hohen Drehmoments für den Anwender im Verlauf der Anwendung zu vermeiden.

6. Medizinische Antriebseinheit (1) der Handheld-Bauart nach Anspruch 5,
**dadurch gekennzeichnet, dass** eine Länge bzw. Dauer der durch die Steuer- oder Regeleinrichtung bewirkten Drehmomentbegrenzung von der Zeit abhängt, die der Anwender für eine sichere Anwendung der medizinischen Antriebseinheit (1) benötigt, um Verletzungen und eine außer Kontrolle des Anwenders geratene Anwendung zu vermeiden.

7. Medizinische Antriebseinheit (1) der Handheld-Bauart nach einem der vorstehenden Ansprüche 1 bis 6, **dadurch gekennzeichnet, dass** der Motor (2a) ein kommutierter Elektromotor ist.

8. Medizinische Antriebseinheit (1) der Handheld-Bauart nach einem der vorstehenden Ansprüche 1 bis 7, **dadurch gekennzeichnet, dass** die Kick-Back-Control bei einer Erfassung einer vorbestimmten Antriebseinheit- oder Haltebewegung-Zbeschleunigung oder eines vorbestimmten Antriebseinheits- oder Haltebewegungs-Zbeschleunigungsbetrags ein Abschalten und / oder Abbremsen und / oder ein Ansteuern des Motors (2a) in entgegenwirkende Richtung und / oder eine Änderung der Drehmoment-Übertragungsfähigkeit eines dem Motor (2a) nachgeschalteten Getriebes (5) automatisch bewirkt.

## Claims

1. A medical drive unit (1), preferably of the handheld type for receiving and rotatingly driving of a medical, preferably surgical tool (6), by means of a motor (2a), preferably an electric motor, housed in the drive unit (1) and operable by means of a controller or regulation unit, comprising a sensor device (3) directly or indirectly detecting or determining a movement or acceleration of the drive unit (1) or a holding movement or holding acceleration of a user, especially the holding hand of the user, which is triggered by the motor (2a) corresponding to its power applied to the tool (6) and having a kickback control unit or tool, which automatically effects a reduction of the driving power or turning off of the motor (2a) upon detection of a predetermined drive-unit or holding movement/acceleration or a predetermined drive-unit or holding-movement/acceleration amount, **characterized in that** the controller or regulation unit has a learning function, which is adapted to identify a current torque for a current application and/or a current user and to determine the currently identified torque as the maximum permissible torque at which the kickback controller has effected the reduction of the driving power or turning off of the motor (2), wherein torques are limited to the maximum permissible torque during the further course of the application.

2. The medical drive unit (1) of the handheld type according to claim 1,
**characterized in that** the sensor device (3) is an inertial sensor, preferably a gyroscope and/or an acceleration sensor, which detects, via three axes (X, Y, Z), a movement of the medical drive unit (1) in the movement direction of the medical tool (6) clamped in the medical drive unit (1).

3. The medical drive unit (1) of the handheld type according to one of claims 1 or 2,
**characterized in that** the control or regulation unit determines based on the rate of change of the movement of the medical drive unit (1) in the movement direction of the medical tool (6) clamped in the medical drive unit (1) whether an increased torque input or an increased rise in torque is present.

4. The medical drive unit (1) of the handheld type according to claims 2 and 3,
**characterized in that** the inertial sensor and the control or regulation unit are integrated in a grip (2) of the drive unit (1) or in an accumulator (4), preferably in an accumulator head, which is further preferably removably inserted in the grip (2) of the drive unit (1).

5. The medical drive unit (1) of the handheld type according to claim 3,
**characterized in that** the torque limiting of the medical drive unit (1) is customized to the application and the user in a permanently adaptable manner by the control or regulation unit, wherein a torque of the medical drive unit (1) is preferably continuously adapted or being adapted by the control or regulation unit to the torque that is currently maximally usable for the user, in order to avoid the occurrence of excessive torque for the user in the course of the application.

6. The medical drive unit (1) of the handheld type according to claim 5,
**characterized in that** a length or duration of the torque limiting effected by the control or regulation unit depends on the time which the user needs for stabilizing the medical drive unit (1).

7. The medical drive unit (1) of the handheld type according to one of the preceding claims 1 to 6, **characterized in that** the motor (2a) is a commutated electric motor.

8. The medical drive unit (1) of the handheld type according to one of the preceding claims 1 to 7, **characterized in that** the kickback control automatically effects turning off and/or decelerating and/or driving the motor (2a) in the reverse direction and/or a change in the torque-transmission capability of a gear (5) connected downstream of the motor (2a) upon detection of a predetermined drive-unit or holding movement/acceleration or of a predetermined drive-unit or holding-movement/acceleration amount.

## Revendications

1. Unité d'entraînement médicale (1), de préférence du type portatif, destinée à recevoir et à entraîner en rotation un outil médical, de préférence chirurgical (6), au moyen d'un moteur (2a), de préférence un moteur électrique, logé dans l'unité d'entraînement (1) qui peut être actionné par un appareil de commande ou de régulation, avec un dispositif de capteur (3) pour la détection ou la détermination directe ou indirecte d'un mouvement ou d'une accélération de l'unité d'entraînement (1) ou d'un mouvement de maintien ou d'une accélération de maintien d'un utilisateur, lequel mouvement ou laquelle accélération est déclenché(e) par le moteur (2a) en fonction de sa puissance délivrée à l'outil (6) et avec un contrôle de rebond qui, lors de la détection d'une accélération prédéterminée de l'unité d'entraînement ou du mouvement de maintien ou d'une quantité prédéterminée d'accélération de l'unité d'entraînement ou du mouvement de maintien provoque automatiquement une réduction de la puissance d'entraînement ou l'arrêt du moteur (2a), **caractérisée en ce que** l'appareil de commande ou de régulation présente une fonction d'apprentissage qui identifie, pour une utilisation actuelle et/ou un utilisateur actuel, le couple actuel et fixe le couple actuel identifié en tant que couple maximal admissible pour lequel le contrôle de rebond a provoqué la réduction de la puissance d'entraînement ou l'arrêt du moteur (2), dans laquelle des couples sont limités au couple maximal admissible dans le cadre de la suite de l'utilisation.

2. Unité d'entraînement médicale (1) du type portatif selon la revendication 1,
**caractérisée en ce que** le dispositif de capteur (3) est un capteur inertiel, de préférence un gyroscope et/ou un capteur d'accélération, qui détecte, par l'intermédiaire de trois axes (X, Y, Z), un mouvement de l'unité d'entraînement médicale (1) dans la direction de mouvement de l'outil médical (6) serré dans l'unité d'entraînement médicale (1).

3. Unité d'entraînement médicale (1) du type portatif selon l'une quelconque des revendications 1 ou 2, **caractérisée en ce que** l'appareil de commande ou de régulation détermine s'il y a une entrée de couple accrue ou une augmentation de couple accrue l'aide du taux de variation du mouvement de l'unité d'entraînement médicale (1) dans la direction de mouvement de l'outil médical (6) serré dans l'unité d'entraînement médicale (1).

4. Unité d'entraînement médicale (1) du type portatif selon les revendications 2 et 3,
**caractérisée en ce que** le capteur inertiel et l'appareil de commande ou de régulation sont intégrés dans une poignée (2) de l'unité d'entraînement (1) ou dans un accumulateur (4), de préférence dans la tête d'accumulateur, qui est en outre insérée, de préférence de manière amovible, dans la poignée (2) de l'unité d'entraînement (1).

5. Unité d'entraînement médicale (1) du type portatif selon la revendication 3,
**caractérisée en ce que** la limitation de couple de l'unité d'entraînement médicale (1) peut être adaptée en permanence à l'utilisation et à l'utilisateur par l'appareil de commande ou de régulation, dans laquelle un couple de l'unité d'entraînement médicale (1) est ou sera adapté de préférence en continu au couple maximal utilisable actuellement par l'utilisateur par l'appareil de commande ou de régulation pour éviter l'apparition d'un couple trop élevé pour l'utilisateur au cours de l'utilisation.

6. Unité d'entraînement médicale (1) du type portatif selon la revendication 5,
**caractérisée en ce qu'**une longueur ou une durée de la limitation de couple provoquée par l'appareil de commande ou de régulation dépend du temps dont l'utilisateur a besoin pour une utilisation sûre de l'unité d'entraînement médicale (1) pour éviter des blessures et une utilisation échappant au contrôle de l'utilisateur.

7. Unité d'entraînement médicale (1) du type portatif selon l'une quelconque des revendications précédentes 1 à 6, **caractérisée en ce que** le moteur (2a) est un moteur électrique commuté.

8. Unité d'entraînement médicale (1) du type portatif selon l'une quelconque des revendications précédentes 1 à 7, **caractérisée en ce que** le contrôle de rebond provoque automatiquement un arrêt et/ou un freinage et/ou une commande du moteur (2a) dans le sens opposé et/ou une modification de la capacité de transmission de couple d'un engrenage (5) monté en aval du moteur (2a) lors d'une détection d'un mouvement de maintien/d'une accélération prédéterminé de l'unité d'entraînement ou d'un mouvement de maintien/d'une quantité prédéterminé d'accélération de l'unité d'entraînement ou du mouvement de maintien.
